# EUROPEAN PATENT SPECIFICATION

(11) **EP 3 649 158 B1**
(45) Date of publication and mention of the grant of the patent: **07.05.2025**
(21) Application number: 18752881.5
(22) Date of filing: 14.06.2018
(51) Int. Cl.: C07K 16/30, A61K 39/39, C07K 19/00, C07K 14/31

(54) **SPLIT SUPERANTIGENS AND THEIR USE FOR IMMUNOTHERAPY**
GESPALTENE SUPERANTIGENE UND IHRE VERWENDUNG ZUR IMMUNTHERAPIE
SUPERANTIGÈNES SÉPARÉS ET LEUR UTILISATION EN IMMUNOTHÉRAPIE

(30) Priority: 05.07.2017 SI 201700202
(43) Date of publication of application: 13.05.2020
(73) Proprietor: Kemijski Institut, 1000 Ljubljana (SI)
(72) Inventor: JERALA, Roman, 1000 Ljubljana (SI); GOLOB URBANC, Anja, 4000 Kranj (SI)
(74) Representative: Weickmann & Weickmann PartmbB
(86) International application number: PCT/SI2018/050019
(87) International publication number: WO 2019/009819

(56) References cited:
- KELCEY G. PATTERSON ET AL: "Control of Established Colon Cancer Xenografts Using a Novel Humanized Single Chain Antibody-Streptococcal Superantigen Fusion Protein Targeting the 5T4 Oncofetal Antigen", PLOS ONE, vol. 9, no. 4, 15 April 2014 (2014-04-15), pages e95200, XP055156461, DOI: 10.1371/journal.pone.0095200
- FORSBERG G ET AL: "THERAPY OF HUMAN NON-SMALL-CELL LUNG CARCINOMA USING ANTIBODY TARGETING OF A MODIFIED SUPERANTIGEN", BRITISH JOURNAL OF CANCER, NATURE PUBLISHING GROUP, GB, vol. 85, no. 1, 6 July 2001 (2001-07-06), pages 129 - 136, XP001145660, ISSN: 0007-0920, DOI: 10.1054/BJOC.2001.1891
- GIDLF ET AL: "A superantigen-antibody fusion protein for T-cell immunotherapy of human B-lineage malignancies.", BLOOD, vol. 89, no. 6, 1 March 1997 (1997-03-01), pages 2089 - 2097, XP055032303, ISSN: 0006-4971
- YONGSHENG YU ET AL: "Functional Assembly of Protein Fragments Induced by Spatial Confinement", PLOS ONE, vol. 10, no. 4, 15 April 2015 (2015-04-15), pages e0122101, XP055504044, DOI: 10.1371/journal.pone.0122101
- A. E. CAMPBELL ET AL: "Targeting protein function: the expanding toolkit for conditional disruption", BIOCHEMICAL JOURNAL, vol. 473, no. 17, 29 August 2016 (2016-08-29), GB, pages 2573 - 2589, XP055504052, ISSN: 0264-6021, DOI: 10.1042/BCJ20160240
- RISHI RAKHIT ET AL: "Chemical Biology Strategies for Posttranslational Control of Protein Function", CHEMISTRY AND BIOLOGY., vol. 21, no. 9, 1 September 2014 (2014-09-01), GB, pages 1238 - 1252, XP055504045, ISSN: 1074-5521, DOI: 10.1016/j.chembiol.2014.08.011
- LILY ZHANG ET AL: "Assessment of the Functional Regions of the Superantigen Staphylococcal Enterotoxin B", TOXINS, vol. 5, no. 10, 22 October 2013 (2013-10-22), pages 1859 - 1871, XP055504046, DOI: 10.3390/toxins5101859

## Description

### Field of invention

The field of invention is directed at a split superantigen, divided into two fragments that by itself do not exhibit any activity, only upon dimerization they regain T cell activity.

Scope of the invention is a screening method for detection of effective split superantigen designs, where split superantigen fragments are fused with coiled-coil forming peptides.

The present invention relates to the field of cancer immunology. Each split superantigen fragment is fused with antibody or other tumor antigen specific protein, therefore only upon the binding of split superantigen-antibody fusion proteins to the tumor antigen, split superantigen fragments come to proximity and reconstitute into biologically active form that activates T-cell response.

### State of the art

Lately, in the field of cancer therapy, we are following great progress, especially in the development of biological drugs. Nevertheless, cancer in developed countries remains one of the leading causes of death. In a last decade an important approach for fighting cancer became immunotherapy, where the main goal is to activate patient own immune system to specifically recognize and kill tumor cells. Antibody-based therapeutics that target surface antigens expressed on tumor cells, are successfully used for treatment of different types of cancer. Targeted tumor therapy with monoclonal antibodies can prevent the proliferation of tumor cells by blocking specific molecules required for tumor growth or can invoke tumor cell death through mechanisms such as: (i) antibody-dependent cellular cytotoxicity (ADCC), (ii) complement-mediated cytotoxicity (CMC), (iii) antibody-dependent cellular phagocytosis (ADCP) [1].

Although unconjugated monoclonal antibodies have efficacy, clinical studies showed that conjugating cytotoxic agents to monoclonal antibodies enhance their clinical utility [2]. Antibody-drug conjugates (ADC) are class of highly potent biological drugs, composed of an antibody and an effector molecule. The effector domain can be: (i) a cytotoxic or radioactive molecule that kill the tumor cells, (ii) immunotoxin that activates the patient own immune system against tumor [1].

One of the immunotherapy approaches includes superantigens as effector molecules. Superantigens are potent activators of T lymphocytes and can activate up to 20 % of T cells in comparison to peptide antigens that activate only a fraction of T cells (0,001 % or less) [3], [4]. The best characterized are the family of staphylococcal enterotoxins and streptococcal pyrogenic exotoxins secreted by the gram positive bacteria *Staphylococcus aureus* or *Streptococcus pyogenes* [4], [5]. Superantigens do not need to be processed through antigen presenting cells but can directly bind to class II major histocompatibility complex (MHCII) expressed on antigen presenting cells. Once bound to MHC class II, superantigen sequentially binds the T cell receptor (TCR) via the variable region of the TCR β chain [5]. This results in activation of both, as of cytotoxic T cells (CD8+) as of helper T cells (CD4+), including massive release of perforins and cytokines, such as IL-2, IFN-y and TNF-α, causing tumor cell death. The precondition for activating T cells is the superantigen binding to the MHC class II on the target cell [6]. All tumor cells do not express MHC class II, therefore to make superantigens selective for tumor antigens, Dohlsten et al. [7]-[9] conjugated superantigen staphylococcal enterotoxin A (SEA) from *Staphylococcus aureus* with monoclonal antibodies specific for different tumor antigens. Novel superantigen-based tumor therapeutics is cytotoxic for tumor cells that express target antigens, independent of MHC class II.

### Disadvantages of state of the art

Fusion proteins with Fab part of antibody specific for C242 antigen and wildtype SEA have been investigated in clinical trials phase I of colorectal and pancreatic cancer. Most commonly occurred side effects were high fever and hypotension [10]. Toxic side effects probably occur due to high affinity of superantigen to MHC class II expressed on B cells, dendritic cells and macrophages [11]. This cause's systemic cytokine release, therefore due to the limited maximum tolerated dose (MTD) optimal therapeutic response is not reached [12], [13].

To lower the systemic effect of Fab-SAg fusion proteins, the substitution Asp227 to Ala (D227A) was introduced into the SEA moiety, reducing binding activity to MHC class II up to 1000 times without affecting the TCR binding [14], [15]. This point mutation is in the SEA high affinity MHC class II binding site, which interacts with β chain of MHC class II complex in zinc dependent manner. But SEA also contains a low affinity MHC class II binding site that interacts with α chain of MHC class II complex [16].

Recent research is based on antibody-superantigen fusion proteins composed of target seeking moieties, usually antibodies, fused with superantigen (wildtype, mutated or chimeric) [23]. Dohlsten et al. [7]-[9] were first to exploit the conjugates between wildtype superantigen and antibody. For purposes of this first generation of antibody-superantigen fusion proteins, some investigators also used wildtype SEB [17]. Second generation of antibody-superantigen fusion proteins comprise an antibody linked with mutated SEA_{D227A} with reduced binding activity to MHC class II [14], [15]. Erlandsson et al. [13] improved fusion proteins with chimeric superantigen SEA/E-120. In addition to reduced binding activity to MHC class II, this third generation fusion proteins is also expected to be less immunogenic in comparison to wildtype SEA. Although substitution D227A in SEA did reduce the binding affinity to MHC class II, the systemic cytotoxic effect on MHC class II expressing cells was only lowered, not eliminated [15].

### Our solution

The invention is defined by the appended claims. Any other aspects described herein are only provided to illustrate the field of the invention.

To overcome the limitation of previous antibody-superantigen fusion proteins, we designed a new generation of superantigens that are not capable of binding to MHC class II or if they bind, the activation of T cells does not occur. We invented split sperantigens as defined in claims 1-7, where superantigen is split into two fragments, N-terminal fragment and C-terminal fragment, each by itself inactive, until fragments came into close proximity, dimerize and reconstitute into a biologically active form capable of activating T cell response.

We also developed a screening method as defined in claim 11, that enables us to easily detect effective split superantigen designs. Split superantigen fragments were fused with polypeptides and superantigen regain its biologic activity only when split fragments were fused with coiled-coil forming peptides, meanwhile split fragments fused with non-coiled-coil forming polypeptides, did not regain its activity.

The split superantigen according to the invention is additionally fused with target seeking moiety, namely an antibody or antibody fragment specific for cell surface proteins, in particular specific for tumor antigen, for purposes of cancer immunotherapy. Split superantigen design that proved efficacy when fused with coiled-coil forming peptides, was fused with antibody specific for tumor antigen. Hence only when antibody bound to the tumor antigen, split superantigen fragments came to proximity, dimerized and folded into biologically active form that activated T cell response, which lead to tumor cell death.

### Brief description of the drawings

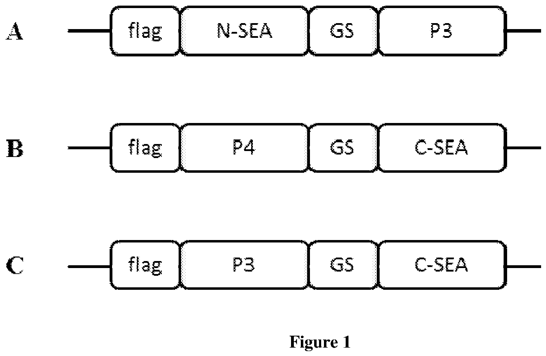

**Figure 1****.** Scheme of split superantigen fused with polypeptides. All gene constructs contain FLAG tag on the N-terminal end. Split superantigen fragments, N-terminal fragment (N-SEA) or C-terminal fragment (C-SEA), are fused with polypeptides P3 or P4 trough glycine-serine (GS) peptide linker.

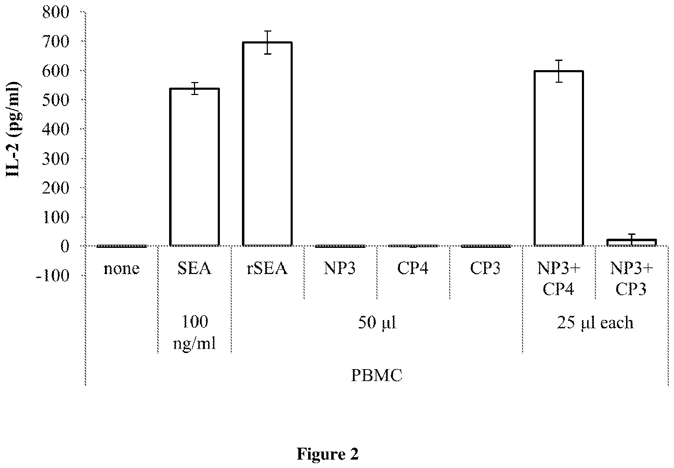

**Figure 2****.** PBMC stimulation with split superantigen fused to polypeptides. 24 hours after seeding, PBMC were stimulated with 100 ng/ml commercially available superantigen (SEA) or with supernatant collected from HEK293T cells containing 50 µl of recombinant wildtype SEA (rSEA), 50 µl of split superantigen fused with polypeptides P3 or P4 (NP3, CP3, CP4), respectively, or with 25 µl of each split superantigen fragments fused with polypeptides P3 or P4, combined. After 24 hour incubation at 37°C, supernatant was collected and the production of human IL-2 was measured by ELISA assay.

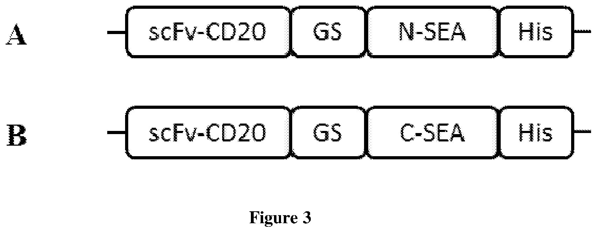

**Figure 3****.** Scheme of split superantigen fused with antibodies. All gene constructs contain His tag, composed of ten histidine amino residues, on the C-terminal end. Single chain variable fragment against tumor antigen CD20 (scFv-CD20) is fused with split superantigen fragments, N-terminal fragment (N-SEA) or C-terminal fragment (C-SEA), trough glycine-serine (GS) peptide linker.

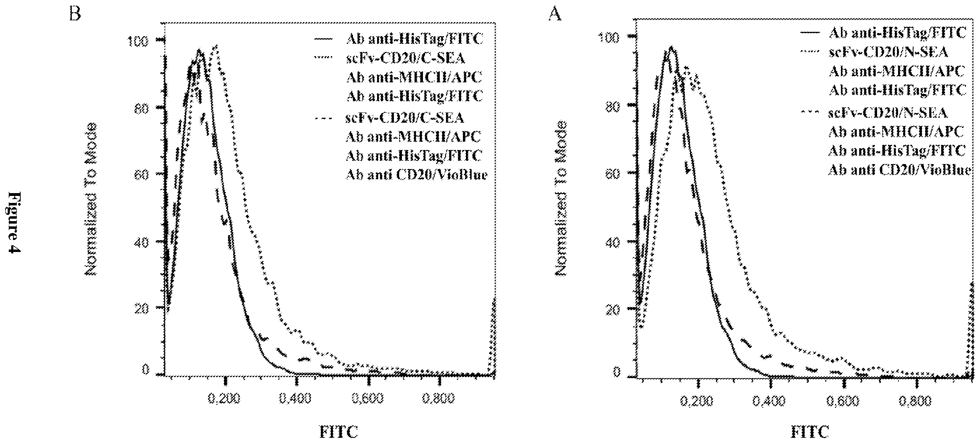

**Figure 4****.** Binding assay. Raji cells were incubated with antibodies against HLA-DR,DP,DQ labeled with APC and/or antibodies against CD20 labeled with VioBlue. After 10 minutes of incubation in dark at 4°C, cells were washed. Cells were then incubated with 150 nM fusion proteins scFv-CD20/N-SEA **(****Figure 4A****)** or scFv-CD20/C-SEA **(****Figure 4B****)** for 1 hour at 37°C. After washed with PBS, cells were incubated 10 minutes in the dark at 4°C, with antibodies against His tag labeled with FITC. Binding of His-tagged fusion proteins scFv-CD20/N-SEA and scFv-CD20/C-SEA to target antigen CD20 expressed on lymphoma cell line Raji was detected with flow cytometry.

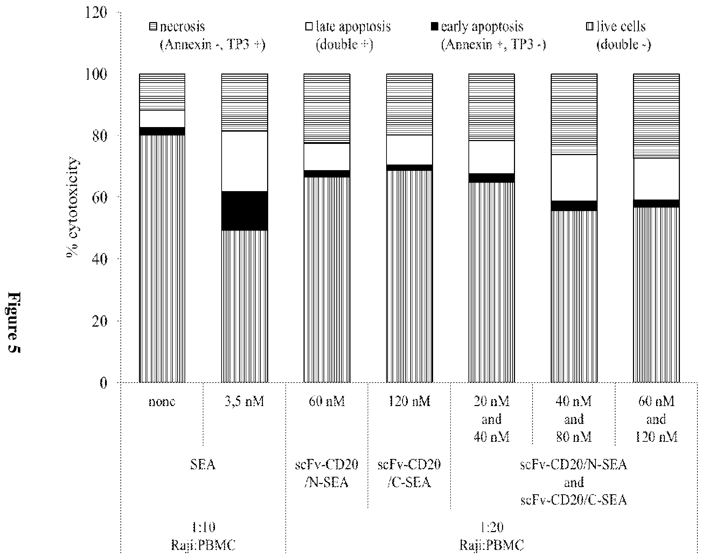

**Figure 5****.** Cytotoxicity assay. Raji cells stained with CFSE were incubated with commercially available SEA (Sigma-Aldrich) and with different concentrations of fusion proteins scFv-CD20/N-SEA and/or scFv-CD20/C-SEA. After 1h at 37°C effector cells (PBMC) were added at ratios 1:10 or 1:20. Viability of tumor cells Raji was determined after 48h at 37°C. Raji cells were stained with viability dyes, Annexin V-Pacific Blue (stain apoptotic cells) and TO-PRO-3 (stain necrotic cells), and analyzed on flow cytometry.

### Detailed description

Unless defined otherwise, all technical and scientific terms used herein possess the same meaning as it is commonly known to experts in the field of invention. The terminology to be used in the description of the invention has the purpose of description of a particular segment of the invention and has no intention of limiting the invention. All publications mentioned in the description of the invention are listed as references. In the description of the invention and in the claims, the description is in the singular form, but also includes the plural form, what is not specifically highlighted for ease of understanding.

The present invention describes a new type of split proteins as defined in claims 1-7. The basis of the invention is the surprising discovery that toxins called superantigens can be divided into two fragments, each by itself not biologically active, they only acquire activity when dimerizing upon binding to cells that express tumor antigen on the surface.

One particular embodiment of the invention is a screening method as defined in claim 11, for the detection of effective split superantigen designs. Split superantigen design is a challenging task, since it is impossible to predict the desirable split sites that would ensure that each split fragment by itself would not exhibit any activity, that the fragments would not reassemble spontaneously, and ideally, that reassembled split protein would have the activity comparable to wildtype protein. In most cases split proteins completely lose their biological activity or their activity is decreased in comparison to unsplit parent protein [18]. For detection of effective split protein designs it is desirable to have an easily measurable read out, as in case of for example split green fluorescent protein [19], where the read out is fluorescence. Depending on the fluorescence intensity it can be determined whether the biological activity of reassembled split protein is comparable to the activity of the unsplit parent protein, meanwhile in case of split superantigens, the read out is more complex. We establish a method for detection of effective split superantigen reassembly, based on split protein fusion with polypeptides. Split superantigen fragments fused with coiled-coil forming polypeptides are reassembled into a biologically active form meanwhile split superantigen fragments fused with polypeptides that do not form coiled-coil do not reassemble. Efficacy of split superantigen is determined based on biologic activity of reassembled protein. In order to monitor the biologic activity of split superantigen, human peripheral blood mononuclear cells (PBMC) were stimulated with fusion proteins (Figure 1) and the release of human cytokine IL-2 was detected with ELISA assay. On the basis of IL-2 release we determined whether T cells were activated. The effective split superantigen variant was the one that activated T cells only when split superantigen was fused with coiled-coil forming polypeptides and did not activate T cell response when split superantigen was fused with polypeptides that do not form coiled-coil (Figure 2).

Further, invention is based on the discovery, that split superantigen can be fused with target seeking moiety, namely an antibody or fragment thereof, specific for tumor antigen, which represents a new tool for treatment in the field of cancer immunotherapy. The binding of two antibodies specific for tumor antigens fused with split superantigen fragments, brings the inactive split fragments into close proximity so they can reassemble into biologically active form, cytotoxic to tumor cells expressing target antigen. The main advantage of the approach is the specificity only for tumor cells without affecting healthy cells expressing MHC class II, which, unlike the standard approach with wildtype superantigen, completely eliminates the toxic systemic effect of superantigen. At the same time, the invention further allows each split superantigen fragment to be coupled to an antibody specific for different tumor antigen, thereby increasing the selectivity of tumor cell recognition.

For illustrative purposes, this document also describes split superantigens that are fused to only a polypeptide or only an antibody. According to the invention, however, both coiled-coil forming polypeptides and antigens or fragments thereof specific for cell surface proteins are fused to a split superantigen, as defined in the claims.

### Definitions

The terms »homologue« and »orthologue« refer to polypeptides, originating from the same or different organism. The term »homologous« also refers to mutated protein segments, where the mutations have a minimal effect on the structure or function of the polypeptide. The term »mutant« refers to a polypeptide, differing from the native protein polypeptide in at least one amino acid.

The term »superantigen« refers to toxins from family of staphylococcal enterotoxins or streptococcal pyrogenic exotoxins secreted by the gram positive bacteria *Staphylococcus aureus* or *Streptococcus pyogenes,* their homologues, orthologues and mutants with preserved or enhanced basic function of superantigens.

The term »split superantigen« refers to complementary superantigen fragments, each by itself biologically inactive. Superantigen is split into two parts, an N-terminal fragment and a C-terminal fragment, that do not reassemble into an active form spontaneously. Split superantigen fragments reassemble into biologically active form only when fused with coiled-coil forming peptides or when fused with antibody bind to tumor antigens. Split site can be located at any site in the superantigen, preferably in unstructured areas. At the same time, the number of overlapping amino acids between the N-terminal fragment and the C-terminal fragment of superantigen fragment is arbitrary.

The term »biologically active form« as used herein, refers to activity a superantigen may exhibit and means it is capable of binding to both MHC complex II and to the TCR which leads to activation of immune response.

The term »fragment« of molecule such as protein or nucleic acid refers to part of amino acid sequence or nucleic acid sequence.

The term »polypeptide« as used herein, refers to polypeptides that form specific dimers called coiled-coils only with its partner. The two polypeptides constituting the orthogonal pair can form parallel homodimers, antiparallel homodimers, or parallel heterodimers. Orthogonality in this case means that only one combination of two polypeptides forms a coiled-coil.

The term »coiled-coil« refers to secondary structure formed by pairs of polypeptides when dimerized. For the coiled-coil formation, it is essential that the polypeptide contains a distinct amino acid sequence. One turn in coiled-coil consists of 3,5 amino acid residues, therefore 2 turns represent about 1 nm long heptade. In the primary peptide structure, specific amino acid residues known to those skilled in the art are present at precisely determined sites in heptade repeat (sites a-b-c-d-e-f-g).

The term »antibody« refers to an immunoglobulin molecule, which is able to specifically bind to a target antigen. In the description of the invention, the term »antibody« is intendent to refer broadly to any immunologic binding agent such as polyclonal antibodies, monoclonal antibodies or antigen binding parts of the antibodies (for example Fab(ab)₂, Fab, Fv, scFv) known to those skilled in the art. The term »antibody« refers to any target seeking moieties, which are specific for tumor antigens, but are not limited to them. In addition to tumor specific antibodies, it is possible to use antibodies against different antigenic determinants, such as, for example, antigens involved in the development of autoimmune, viral diseases.

Superantigen fragments are linked together with polypeptides or antibodies by »peptide linker«, which is any polypeptide of any length and any amino acid sequence. The term »peptide linker« refers to amino acid sequences with the function of separating individual domains of a chimeric protein and to enable their proper spatial orientation.

The term »tag peptide« refers to amino acid sequences, added to a protein for simplified purification, isolation or detection.

The position of signal sequences, linker peptides and tag peptides can be arbitrary, although they should allow functional expression of the protein, while also preserving the function for which these sequences were selected, what is known to those skilled in the art.

The term »constitutive promoter« refers to a nucleotide sequence in DNA that provides a continuous transcription of structural genes, and its location and sequence is known to those skilled in the art. The term »constitutive promoter« refers to a non-regulated promoter that allows a continuous expression of its associated gene. Suitable nucleotide sequences of constitutive promoters are known to those skilled in the art and described in the prior art.

The terms »promoter«, »teminator«, »protein«, »DNA« are generally known to persons skilled in the art and are used as expected.

The term »expression vector« refers to circular or linear DNA plasmids or viral DNA, containing operons listed in the invention and the necessary elements for expression in prokaryotic or eukaryotic cells, which are known to persons skilled in the art. Bacterial vectors contain bacterial control elements, a bacterial replication origin and an antibiotic resistance operon for selection of successfully transformed bacteria. Eukaryotic vectors contain, in addition to a bacterial replication origin, appropriate eukaryotic control elements, and appropriate antibiotic resistance operons for selection of successful bacterial transformation and/or successful eukaryotic transfection.

Embodiments of the invention can be used in prokaryotic as well as in eukaryotic organisms and cell lines using recombinant DNA technology. Methods involving an intervention with a living human or animal body are not encompassed by the scope of the invention. The basic difference is the use of appropriate nucleotide sequences in the promoter and the terminator known to those skilled in the art to ensure the structural gene transcription. One method of preparing fusion proteins is also chemical conjugation, wherein various heterobifunctional or heterobifunctional cross-linkers known to those skilled in the art are used to link the superantigen fragment with polypeptides or antibodies.

Transfer of DNA into host cells is performed with conventional methods well known to persons skilled in the arts, such as transformation, transduction or transfection, including: chemical transfer, electroporation, microinjection, DNA lipofection, cell sonication, gene bombarding, viral DNA transfer etc.

DNA transfer can be either transient or stable. »Transient transfer« refers to transfer of DNA in a vector that does not undergo chromosomal insertion. »Stable transfer« refers to insertion of DNA into the host genome. DNA transfer to a cell line with a previous stable insertion can be controlled with the presence of markers. »Markers« refer to antibiotic or chemical resistance and can be included in the vector or present on a separate vector.

Therefore, one aspect of the present invention refers to split superantigen as defined in claims 1-7, wherein the superantigen is divided into two non-functional fragments that only in close proximity and when fused with coiled-coil forming peptides and fused with antibodies specific to tumor antigens, dimerize and form a biologically active superantigen. Split superantigen fragments are linked with the same or different proteins or polypeptides that, when bound to the target cell, induce the dimerization of inactive split superantigen fragments into the active superantigen. Proteins or polypeptides that trigger the dimerization of inactive split superantigen fragments into the active superantigen are identical or different and recognize the cell proteins expressed on the surface of the target cell, namely antibodies or fragments of antibodies that recognize the surface proteins of the cells. These proteins or polypeptides preferably recognize the surface proteins on tumor cells.

Furthermore, the invention also relates to a screening method as defined in claim 11, for detection of functional split superatnigens, wherein (a) combination of split superantigen fused with proteins that enable dimerization is provided and is preferably controlled and mediated chemically or by the presence of cells, wherein the protein that allows dimerization is selected from a coiled-coil forming polypeptide and an antibody or fragment thereof, (b) split superantigen or cells expressing split superantigens is added to the reporter cells selected from any of the cell lines that are sensitive to the presence of active superantigen, preferably PBMC, and (c) measurement of superantigen activation by usage of reporter cells.

The present invention will now be further described. Examples of implementations described in detail below are conceived to best describe the invention. These descriptions are not intended to limit the field of the invention or its applicability but serve to better demonstrate the invention and its applicability.

### Examples

### Example 1. Preparation of DNA constructs for the fusion proteins according to the invention

For the preparation of DNA constructs the inventors used methods of molecular biology, such as: chemical transformation of competent E. coli cells, DNA plasmid isolation, polymerase chain reaction (PCR), PCR ligation, determination of nucleic acid concentration, agarose gel electrophoresis of DNA, isolation of DNA fragments from agarose gels, chemical synthesis of DNA, DNA digestion with restriction enzymes, digestion of plasmid vectors, ligation of DNA fragments, purification of plasmid DNA in larger quantities. Molecular cloning procedures are well known to the experts in the field and are described in details in molecular biology handbook [20].

All work was performed with sterile techniques, which are well known to persons skilled in the art. All plasmids, completed constructs and partial constructs were transformed into bacteria *E. coli* with chemical transformation. Plasmids and DNA constructs were transfected into HEK293T cell lines using commercially available transfection reagent polyethylenimine.

All DNA constructs have been prepared using techniques and methods known in the art. DNA constructs were inserted into appropriate plasmids suitable for eukaryotic or prokaryotic expression systems. Vectors used were commercially available, carrying all necessary features such as antibiotic resistance, origin of replication and multiple cloning site known to the experts in the field. The inventors confirmed adequacy of nucleotide sequences by sequencing and restriction analysis.

### Example 2. Detection of functional split superantigen by a screening method based on split proteins fused with polypepdites

### a) Design of split superantigen fused with polypeptides

In order to illustrate the invention well characterized staphylococcal enterotoxin A (SEA), from *Staphylococcus aureus,* was used as superantigen. Superantigen SEA was split into two fragments, an N-terminal fragment (1-120 amino acid residues of SEA; SEQ ID: 1) and a C-terminal fragment (112-233 amino acid residues of SEA; SEQ ID NO: 112); which overlap in nine amino acid residues.

Both split SEA fragments were fused with polypeptides via the glycine-serine peptide linker (SEQ ID: 3). The orthogonal pair of polypeptides P3 (SEQ ID: 4) and P4 (SEQ ID: 5) [21] were selected as the most suitable dimerizing polypeptides to form coiled-coil.

Split superatnigens in fusion with polypeptides were prepared using recombinant DNA technology. Mammalian expression system, a human cell line HEK293T, was used. The methods and techniques of culturing cell cultures are well known to persons skilled in the art, therefore they are only briefly described in order to illustrate the embodiment. Cell line HEK293T was cultured at 37 °C and 5% C0₂. DMEM medium supplemented with 10% FBS, which contains all the necessary nutrients and growth factors, was used for cell culturing. Once the cell culture reached an appropriate density, cells were subcultured into a new culture vessel and/or diluted. For the application of cells in experiments the number of cells was determined with a hemocytometer. A day before transfection cells were seeded onto 6 well plates. Seeded plates were incubated at 37 °C and 5% C0₂, until the cells reached the appropriate confluence for the transfection. Transfection was preformed according to the manufacturer's protocol.

The HEK293T cells were transfected with commercially available plasmid pFLAG-CMV containing DNA insert coding for the wildtype SEA or for the selected split superantigen fragments in fusion with the polypeptides. The scheme of the prepared fusion proteins is shown in Figure 1. Plasmid pFLAG-CMV contains a signal sequence for the secretion of proteins from cells that allowed the secretion of fusion proteins into the medium. Three to four days after the transfection, the medium was collected and the presence of fusion proteins in the HEK293T cell supernatant was confirmed with Western blotting.

### b) Testing the efficacy of split superantigen

With this embodiment, it has been shown that split superantigen has been prepared which becomes biologically active only when fused with coiled-coil forming polypeptides (Figure 2).

To prove the biologic activity of selected split superantigen design, PBMC were stimulated with HEK293T cell supernatant containing prepared fusion proteins (Figure 1). The methods and techniques of culturing cell cultures are well known to persons skilled in the art, therefore they are only briefly described in order to illustrate the embodiment. PBMC was obtained from the blood samples of healthy volunteers and were isolated according to the standard protocol [22]. The PBMC were cultured at 37 °C and 5% C0₂ and RPMI medium supplemented with 10% FBS, which contains all the necessary nutrients and growth factors, was used. The number of cells was determined with a hemocytometer. PBMC were seeded (5*10⁴ cells/well) onto 96 well plate and after 24 hours stimulated with 100 ng/ml of commercially available SEA (Sigma-Aldrich), 50 µl of HEK293T supernatant containing recombinant wildtype SEA (rSEA) or 50 µl of split superantigen fused with polypeptides P3 or P4 (NP3, CP3, CP4), respectively, or simulated with 25 µl of each split superantigen fragments fused with polypeptides P3 or P4, combined. After 24 hour incubation at 37°C, supernatant was collected and the production of human IL-2, as an indicator of T-cell activation, was measured by commercially available ELISA assay.

Figure 2 show that each split superantigen fragment fused with P3 or P4 by itself does not cause T cell activation. Activation of T cells also does not occur when both split superantigen fragments are fused with P3 polypeptide, because combination of only P3 polypeptides does not dimerize and form coiled-coil. Thus, when split superantigen fragments are fused as with P3 and P4 polypeptides, coiled-coil is formed, split superantigen is reconstituted and T cells are activated. With this embodiment, it has been shown that we are able to design a split superantigen, capable of activating T cell response only when fused with coiled-coil forming polypeptides, meanwhile in fusion with polypeptides that do not form coiled-coil, activation of T cells do not occur. At the same time, we have shown that the split superantigen fragments cannot spontaneously reassemble into biologically active form.

### Example 3. Use of split superantigen for cancer immunotherapy

### a) Design of split superantigen fused with antibodies

To illustrate the use of the invention for the purpose of cancer immunotherapy, the N-terminal superantigen SEA fragment (N-SEA, 1-120 amino acid residues of SEA; SEQ ID: 1) and C-terminal superantigen SEA fragment (C-SEA, 112-233 amino acid residues of SEA; SEQ ID NO: 112), which overlap in nine amino acid residues, were linked with single chain variable fragment specific for tumor antigen CD20 (scFv-CD20) (SEQ ID: 6), through glycine-serine peptide linker (SEQ ID: 3).

Fusion proteins between split superantigen and scFv were prepared in the prokaryotic expression system using recombinant DNA technology. Methods and techniques for the production, isolation and refolding of recombinant proteins are well known to experts in the field and are here explained only indicative, in terms of clarifying the implementation of the embodiment.

*E.coli* strain BL21 (DE3) was transformed with a commercially available plasmid pET19b, whit inserts coding for split superantigen fragment N-SEA or C-SEA in fusion with scFv-CD20. Scheme of the fusion proteins scFv-CD20/N-SEA and scFv-CD20/C-SEA is shown in figure 3. Transformed bacterial cells were grown on a solid LB medium containing ampicillin (50 µg/ml). After 16 hours incubation at 37°C, the individual bacterial colonies were put into 100 ml of a liquid medium LB containing ampicillin (50 µg/ml). Cells were incubated overnight with shaking (180 rpm) at 37°C.

Density of the bacterial cells was then determined by measuring the absorbance at a wavelength of 600 nm. Bacterial cells were cultured at 37°C with shaking (180 rpm) in 500 ml of the liquid medium LB containing ampicillin (50 µg/ml) at the final density of the cells that corresponded to an absorbance value of A₆₀₀ = 0,1, until the value of A₆₀₀ = 0,8-1 was reached. Then the 0,2 mM IPTG was added to induce the production of recombinant fusion proteins. After 4 hours of incubation under unchanged conditions, the bacterial culture was centrifuged (10 minutes, 5000 rpm, 4°C) and supernatant was discarded. Cell pellet was resuspended in lysis buffer (20 mM Tris-HCl, 200 mM NaCl, 1 mg/ml lysozyme, 1 mM MgCl₂, 1:10 000 V/V benzonase (DNase), 1:500 V/V a mixture of protease inhibitors, 10 mM imidazole, 10% glycerol) and then incubated for 1 hour at room temperature with occasional shaking. Cell lysate was centrifuged (15 minutes, 15 000 rpm, 4°C), supernatant was discarded and pellet with inclusion bodies was stored.

Recombinant fusion proteins containing a histidine marker sequence that were produced in bacteria in the form of inclusion bodies were isolated on chelating chromatography (NiNTA agarose column) under the denaturing conditions. The inclusion bodies were washed twice with buffer (10 mM Tris-HCl, 0 1% DOC, pH 8). After each wash, the inclusion bodies were centrifuged for 15 minutes at 15 000 rpm at 4°C. Bacterial inclusion bodies were solubilized in the binding buffer (6 M GvdHCl, 100 mM NaH2PO4, 10 mM Tris-HCl, pH 8) and purified by NiNTA column that was equilibrated with the same buffer. After solubilized inclusion bodies were applied on the column, the column was incubated overnight at 4°C with shaking, followed by washing of the column with the binding buffer alone, subsequently followed by washing with binding buffer containing 10 mM, 20 mM ter 50 mM imidazole until the absorbance measured at the wavelength of 280 nm in the caught fraction was lower than 0,02. Fusion proteins were eluted wit elution buffer ((6 M GvdHCl, 100 mM NaH₂PO₄, 10 mM Tris-HCl, 300 mM imidazole, pH 8).

The eluted protein was dissolved in the binding buffer to a final concentration of 20-30 µg/ml ad refolding with dialysis was performed. The diluted protein was dialyzed at 4°C three times against 2 L of 20 mM Tris-HCl, 150 mM NaCl, pH 7^{·}5. After dialysis, the refolded protein was concentrated using commercially available concentrated falcons, and the purity of the isolated protein was confirmed by polyacrylamide electrophoresis (Coomassie staining) and Western blotting.

### b) Binding of antibody-superantigen fusion proteins on tumor antigens on cells

To confirm the binding of fusion proteins scFv-CD20/N-SEA and scFv-CD20/C-SEA on target CD20 antigen expressed on B cells, Raji human lymphoma cell line was used as a target cells. The methods and techniques of culturing cell cultures are well known to persons skilled in the art. Raji cells were cultured at 37°C and 5% CO₂ in RMPI supplemented with 10% FBS that contains all the necessary nutrients and growth factors. Once the cell culture reached an appropriate density, cells were diluted and/or used in experiment setup. The number of cells was determined with a hemocytometer. 10⁷ Raji cells were resuspended in 80 µl PBS and, according to the manufacturer's protocol, a blocking reagent FcR and antibodies anti-HLA-DR, DP, DQ labeled with APC and/or antibodies anti-CD20 labeled with VioBlue were added. After 10 minutes incubation at 2-8°C in dark, the cells were washed with PBS and fusion proteins scFv-CD20/N-SEA or scFv-CD20/C-SEA were added at a concentration of 150 nM. Mixture of cells and fusion proteins was incubated for 1 hour at 37°C, then washed with PBS, and incubated for 10 minutes at 2-8°C in dark with antibodies anti-His tag labeled with FITC. Binding of fusion proteins scFv-CD20/N-SEA or scFv-CD20/C-SEA containing His tag on tumor antigen CD20 was analyzed by flow cytometry according to techniques and methods generally known to person skilled in the art.

As seen on figure 4, both fusion proteins scFv-CD20/N-SEA and scFv-CD20/C-SEA at a concentration of 150 nM are bound to target CD20 antigen. In all cases, Raji cells were incubated with an antibody anti-HLA-DR, DP, DQ, thereby confirming that the fusion proteins were bound to target cells via scFv-CD20 and not via superantigen fragments to the MHC class II complex. In addition, it has been shown that in the case of occupancy of targeted sites with commercially available antibody anti-CD20, the binding of fusion proteins to the CD20 antigen significantly decreases.

### c) Cytotoxicity assay

When fusion proteins bind to tumor antigens trough antibodies, the split superantigen fragments should come close enough together to reassemble into a biologically active form, recognized by TCR expressed on T cells and consequently cause tumor cell death. In this embodiment, cytotoxicity of the split superantigen fused with antibodies against tumor antigen, was determined by flow cytometry.

Human lymphoma Raji cell line was used as a target tumor cell line. Raji cells were stained with carboxyfluorescein succinimidyl ester (CFSE), according to the manufacturer's protocol, to differ between target and effector cells. Then cells were counted with a hemocytometer and seeded onto 24 well plate (5*10⁴ cells/well). After 1 hour incubation with commercially available SEA (Sigma-Aldrich) and different concentration of fusion proteins scFv-CD20/N-SEA and scFv-CD20/C-SEA (figure 5), effector cells PBMC were added in ratios 1:10 and 1:20. Subsequently 48 hours later at 37°C, cells were stained with Annexin V-Pacific Blue (stains the apoptotic cells) and TO-PRO-3 (stains necrotic cells). Analysis of the viability of Raji tumor cells was performed with flow cytometer by techniques and methods generally known to those skilled in the art.

Figure 5 shows that each fusion protein scFv-CD20/N-SEA or scFv-CD20/C-SEA alone is not cytotoxic for Raji tumor cells. Combination of both fusion proteins scFv-CD20/N-SEA and scFv-CD20/C-SEA is required to assemble the split superantigen into a biologically active form, which causes death of Raji tumor cells.

### Literature

[1] L. M. Weiner, J. C. Murray, and C. W. Shuptrine, "Antibody-Based Immunotherapy of Cancer," Cell, vol. 148, no. 6, pp. 1081-1084, Mar. 2012.
[2] J. S. Kang and M. H. Lee, "Overview of therapeutic drug monitoring.," Korean J. Intern. Med., vol. 24, no. 1, pp. 1-10, Mar. 2009.
[3] T. Proft and J. D. Fraser, "Bacterial superantigens," Clin. Exp. Immunol., vol. 133, no. 3, pp. 299-306, 2003.
[4] J. Fraser, V. Arcus, P. Kong, E. Baker, and T. Proft, "Superantigens - powerful modifiers of the immune system," vol. 6, no. MARCH, pp. 125-132, 2000.
[5] M. D. Baker and K. R. Acharya, "Superantigens: structure-function relationships.," Int. J. Med. Microbiol., vol. 293, no. 7-8, pp. 529-37, Apr. 2004.
[6] M. Dohlsten, G. Hedlund, and T. Kalland, "Staphylococcal-enterotoxin-dependent cell-mediated cytotoxicity," Immunol. Today, vol. 12, pp. 147-149, 1991.
[7] M. DOHLSTEN, G. HEDLUND, E. AKERBLOM, P. A. LANDO, and T. KALLAND, "Monoclonal antibody-targeted superantigens: A different class of anti-tumor agents," Proc. Nati. Acad. Sci. USA, vol. 88, no. October, pp. 9287-9291, 1991.
[8] M. Dohlsten, L. Abrahmsén, P. Björk, P. a Lando, G. Hedlund, G. Forsberg, T. Brodin, N. R. Gascoigne, C. Förberg, and P. Lind, "Monoclonal antibody-superantigen fusion proteins: tumor-specific agents for T-cell-based tumor therapy.," Proc. Natl. Acad. Sci. U. S. A., vol. 91, no. 19, pp. 8945-9, Sep. 1994.
[9] M. Dohlsten, L. Abrahmsén, L. Ohlsson, and P. Lind, "Immunotherapy of human colon cancer by antibody-targeted superantigens," vol. 41, pp. 162-168, 1995.
[10] B. J. Giantonio, R. K. Alpaugh, J. Schultz, C. McAleer, D. W. Newton, B. Shannon, Y. Guedez, M. Kotb, L. Vitek, R. Persson, P. O. Gunnarsson, T. Kalland, M. Dohlsten, B. Persson, and L. M. Weiner, "Superantigen-based immunotherapy: a phase I trial of PNU-214565, a monoclonal antibody-staphylococcal enterotoxin A recombinant fusion protein, in advanced pancreatic and colorectal cancer.," J. Clin. Oncol., vol. 15, no. 5, pp. 1994-2007, May 1997.
[11] H. Borghaei, K. Alpaugh, G. Hedlund, G. Forsberg, C. Langer, A. Rogatko, R. Hawkins, S. Dueland, U. Lassen, and R. B. Cohen, "Phase I dose escalation, pharmacokinetic and pharmacodynamic study of naptumomab estafenatox alone in patients with advanced cancer and with docetaxel in patients with advanced non-small-cell lung cancer.," J. Clin. Oncol., vol. 27, no. 25, pp. 4116-23, Sep. 2009.
[12] M. Dohlsten, T. Kalland, P. Gunnarsson, P. Antonsson, a Molander, J. Olsson, R. d'Argy, L. Ohlsson, M. Soegaard, R. Persson, and T. Brodin, "Man-made superantigens: Tumor-selective agents for T-cell-based therapy.," Adv. Drug Deliv. Rev., vol. 31, no. 1-2, pp. 131-142, Apr. 1998.
[13] E. Erlandsson, K. Andersson, A. Cavallin, A. Nilsson, U. Larsson-Lorek, U. Niss, A. Sjöberg, M. Wallén-Öhman, P. Antonsson, B. Walse, and G. Forsberg, "Identification of the antigenic epitopes in staphylococcal enterotoxins A and E and design of a superantigen for human cancer therapy," J. Mol. Biol., vol. 333, no. 5, pp. 893-905, 2003.
[14] C. Gidlof, M. Dohlsten, P. Lando, T. Kalland, C. Sundstrom, and T. H. Totterman, "A Superantigen-Antibody Fusion Protein for T-Cell Immunotherapy of Human B-Lineage Malignancies," Blood, vol. 89, pp. 2089-2097, 1997.
[15] G. Forsberg, L. Ohlsson, T. Brodin, P. Björk, P. A. Lando, D. Shaw, P. L. Stern, and M. Dohlsten, "Therapy of human non-small-cell lung carcinoma using antibody targeting of a modified superantigen," vol. 85, pp. 129-136, 2001.
[16] L. Abrahmsén, M. Dohlsten, S. Segrén, P. Björk, E. Jonsson, and T. Kalland, "Characterization of two distinct MHC class II binding sites in the superantigen staphylococcal enterotoxin A," Embo J., vol. 14, no. 13, pp. 2978-2986, 1995.
[17] Q. Tong, K. Liu, X.-M. Lu, X.-G. Shu, and G.-B. Wang, "Construction and characterization of a novel fusion protein MG7-scFv/SEB against gastric cancer.," J. Biomed. Biotechnol., vol. 2010, p. 121094, Jan. 2010.
[18] S. S. Shekhawat and I. Ghosh, "Split-protein systems: beyond binary protein-protein interactions.," Curr. Opin. Chem. Biol., vol. 15, no. 6, pp. 789-97, Dec. 2011.
[19] K. P. Kent, W. Childs, S. G. Boxer, and S. U. V, "Deconstructing Green Fluorescent Protein that it can be directly observed by electrospray time-of-flight mass," pp. 9664-9665, 2008.
[20] L. Dong, L.-B. Lv, and R. Lai, Molecular cloning: A laboratory manual, 4th ed., vol. 1, no. 1. 2012.
[21] H. Gradišar and R. Jerala, "De novo design of orthogonal peptide pairs forming parallel coiled-coil heterodimers.," J. *Pept. Sci.,* vol. 17, no. 2, pp. 100-6, Feb. 2011.
[22] Biotec Miltenyi, "Isolation of mononuclear cells from human peripheral blood by density gradient centrifugation," Miltenyibiotec.Com, 2008. [Online]. Available: https://www.miltenyibiotec.com/~/media/Files/Navigation/Research/Stem Cell/SP_MC_PB_density_gradient.ashx.
[23] Patterson et al., "Control of established colon cancer xenografts using a novel humanized single chain antibody-streptococcal superantigen fusion protein targeting the 5T4 oncofetal antigen", PLOS One, vol. 9, no.4, 2014, p. e95200.

## Claims

1. Split superantigen, wherein the split superantigen comprises two non-functional superantigen fragments that only after dimerization, due to the close proximity of both fragments, reassemble into a biologically active form and
wherein the split superantigen fragments are fused with proteins or polypeptides that trigger dimerization, and
wherein the proteins or polypeptides that trigger dimerization are selected from coiled-coil forming polypeptides and antibodies or antibody fragments specific for cell surface proteins, in particular specific for tumor cell surface proteins.

2. Split superantigen according to claim 1, wherein the superantigen originates from the family of superantigens, their homologous, orthologous or mutants with a preserved or improved primary superantigen function, the superantigen preferably being staphylococcal enterotoxin A (SEA) from *Staphylococcus aureus.*

3. Split superantigen according to claim 2, wherein the split superantigen SEA fragments are preferentially N-terminal fragment of superantigen SEQ ID: 1 and C-terminal fragment of superantigen SEQ ID:2.

4. Split superantigen according to claims 1-3, wherein the split superantigen fragments are fused with the same or different proteins or polypeptides, that cause a dimerization of the non-functional split superantigen fragments into biologically active superantigen.

5. Split superantigen according to claims 1-4, wherein the split superantigen fragments are fused with the proteins or polypeptides through gene fusion or chemical conjugation.

6. Split superantigen according to claims 1-5, wherein the proteins or polypeptides that trigger the dimerization of inactive split superantigen fragments into an active superantigen, are the same or different and specifically recognize cell proteins expressed on the surface of target cells, in particular tumor cell surface proteins.

7. Split superantigen according to claims 1-6, wherein the superantigen fragments are expressed and secreted from human cells.

8. DNA coding for the split superantigen according to claims 1-7

9. Use of the split superantigen according to claims 1-7, for the preparation of an active compound, which causes cell death.

10. Split superantigen according to claims 1-6 for use in a method of treating cancer.

11. Screening method for detection of functional split superantigens according to claims 1-6, wherein:
a. superantigen fragments are fused with proteins that allow dimerization, which is preferably controlled and mediated chemically or by the presence of cells, wherein the protein that allows dimerization is selected from a coiled-coil forming polypeptide and an antibody or antibody fragment specific for cell surface proteins, in particular specific for tumor cell surface proteins,
b. superantigen fragments or cells expressing superantigen fragments are added to reporter cells, which are selected from any cell lines sensitive to the presence of active superantigen, preferably PBMC, and
c. analysis of superantigen activation using reporter cells.

## Patentansprüche

1. Spalt-Superantigen, wobei das Spalt-Superantigen zwei nicht-funktionelle Superantigen-Fragmente umfasst, welche sich erst nach Dimerisierung aufgrund der räumlichen Nähe von beiden Fragmenten zu einer biologisch aktiven Form wieder zusammensetzen, und
wobei die Spalt-Superantigen-Fragmente mit Proteinen oder Polypeptiden fusioniert sind, welche Dimerisierung auslösen, und
wobei die Proteine oder Polypeptide, welche Dimerisierung auslösen, ausgewählt sind aus Coiled-Coil-bildenden Polypeptiden und Antikörpern oder Antikörper-Fragmenten, welche für Zelloberflächenproteine spezifisch sind, insbesondere spezifisch für Tumorzelloberflächenproteine.

2. Spalt-Superantigen nach Anspruch 1, wobei das Superantigen aus der Familie der Superantigene stammt, deren Homologen, Orthologen oder Mutanten mit einer konservierten oder verbesserten primären Superantigenfunktion, wobei das Superantigen vorzugsweise Staphylokokken-Enterotoxin A (SEA) von *Staphylococcus aureus* ist.

3. Spalt-Superantigen nach Anspruch 2, wobei die Spalt-Superantigen-SEA-Fragmente bevorzugt N-terminale Fragmente von Superantigen SEQ ID: 1 und C-terminale Fragmente von Superantigen SEQ ID: 2 sind.

4. Spalt-Superantigen nach Ansprüchen 1-3, wobei die Spalt-Superantigen-Fragmente mit gleichen oder verschiedenen Proteinen oder Polypeptiden fusioniert sind, welche eine Dimerisierung der nicht-funktionellen Spalt-Superantigen-Fragmente in ein biologisch aktives Superantigen bewirken.

5. Spalt-Superantigen nach Ansprüchen 1-4, wobei die Spalt-Superantigen-Fragmente mit den Proteinen oder Polypeptiden durch Genfusion oder chemische Konjugation fusioniert sind.

6. Spalt-Superantigen nach Ansprüchen 1-5, wobei die Proteine oder Polypeptide, welche die Dimerisierung von inaktiven Spalt-Superantigen-Fragmenten in ein aktives Superantigen auslösen, die gleichen oder verschieden sind und spezifisch Zellproteine erkennen, welche auf der Oberfläche von Zielzellen exprimiert werden, insbesondere Tumorzelloberflächenproteine.

7. Spalt-Superantigen nach Ansprüchen 1-6, wobei die Superantigen-Fragmente von humanen Zellen exprimiert und sekretiert werden.

8. DNA kodierend für das Spalt-Superantigen nach Anspruch 2.

9. Verwendung des Spalt-Superantigens nach Ansprüchen 1-7 für die Herstellung eines Wirkstoffs, welcher Zelltod verursacht.

10. Spalt-Superantigen nach Ansprüchen 1-6 zur Verwendung in einem Verfahren zur Behandlung von Krebs.

11. Screening-Verfahren zum Detektieren von funktionellen Spalt-Superantigenen nach Ansprüchen 1-6, wobei:
a. Superantigen-Fragmente mit Proteinen fusioniert werden, welche Dimerisierung erlauben, welche vorzugsweise chemisch oder durch die Anwesenheit von Zellen kontrolliert und vermittelt wird, wobei das Protein, welches Dimerisierung erlaubt, ausgewählt ist aus einem Coiled-Coil-bildenden Polypeptid und einem Antikörper oder Antikörper-Fragment, welches spezifisch für Zelloberflächenproteine ist, insbesondere spezifisch für Tumorzelloberflächenproteine,
b. Superantigen-Fragmente oder Zellen, welche Superantigen-Fragmente exprimieren, zu Reporterzellen hinzugefügt werden, welche aus beliebigen Zelllinien ausgewählt werden, welche für die Anwesenheit von aktivem Superantigen empfindlich sind, vorzugsweise PBMC, und
c. Analyse von Superantigen-Aktivierung unter Verwendung von Reporterzellen.

## Revendications

1. Superantigène clivé, où le superantigène clivé comprend deux fragments de superantigène non fonctionnels qui, seulement après la dimérisation, du fait de la proximité immédiate des deux fragments, se réassemblent en une forme biologiquement active et
où les fragments de superantigène clivé sont fusionnés avec des protéines ou des polypeptides qui déclenchent la dimérisation, et
où les protéines ou les polypeptides qui déclenchent la dimérisation sont choisis parmi les polypeptides formant des superhélices et les anticorps ou les fragments d'anticorps spécifiques de protéines de la surface cellulaire, notamment spécifiques de protéines de la surface de cellules tumorales.

2. Superantigène clivé selon la revendication 1, où le superantigène provient de la famille des superantigènes, de leurs homologues, orthologues ou mutants avec une fonction de superantigène primaire préservée ou améliorée, le superantigène étant de préférence l'entérotoxine A staphylococcique (SEA) de *Staphylococcus aureus.*

3. Superantigène clivé selon la revendication 2, où les fragments de superantigène clivé SEA sont préférentiellement le fragment N-terminal de superantigène SEQ ID: 1 et le fragment C-terminal de superantigène SEQ ID: 2.

4. Superantigène clivé selon les revendications 1 à 3, où les fragments de superantigène clivé sont fusionnés avec des protéines ou des polypeptides identiques ou différents, qui provoquent une dimérisation des fragments de superantigène clivé non fonctionnels en superantigène biologiquement actif.

5. Superantigène clivé selon les revendications 1 à 4, où les fragments de superantigène clivé sont fusionnés avec les protéines ou les polypeptides par fusion génique ou conjugaison chimique.

6. Superantigène clivé selon les revendications 1 à 5, où les protéines ou les polypeptides qui déclenchent la dimérisation de fragments de superantigène clivé inactifs en un superantigène actif sont identiques ou différents et reconnaissent spécifiquement des protéines cellulaires exprimées sur la surface de cellules cibles, en particulier des protéines de la surface de cellules tumorales.

7. Superantigène clivé selon les revendications 1 à 6, où les fragments de superantigène sont exprimés et sécrétés par des cellules humaines.

8. ADN codant le superantigène clivé selon les revendications 1 à 7.

9. Utilisation du superantigène clivé selon les revendications 1 à 7 pour la préparation d'un composé actif, qui provoque la mort cellulaire.

10. Superantigène clivé selon les revendications 1 à 6 destiné à être utilisé dans un procédé de traitement du cancer.

11. Procédé de criblage pour la détection de superantigènes clivés fonctionnels selon les revendications 1 à 6, où:
a. des fragments de superantigène sont fusionnés avec des protéines qui permettent la dimérisation, qui est de préférence régulée et médiée chimiquement ou par la présence de cellules, où la protéine qui permet la dimérisation est choisie parmi un polypeptide formant des superhélices et un anticorps ou un fragment d'anticorps spécifique de protéines de la surface cellulaire, en particulier spécifique de protéines de la surface de cellules tumorales,
b. des fragments de superantigène ou des cellules exprimant des fragments de superantigène sont ajoutés à des cellules rapporteuses, qui sont choisies parmi toutes les lignées cellulaires sensibles à la présence de superantigène actif, de préférence des PBMC, et
c. analyse de l'activation du superantigène à l'aide de cellules rapporteuses.
